Europäisches Patentamt

European Patent Office

Office européen des brevets

(11) Numéro de publication: **0 297 972**
**A2**

(12) **DEMANDE DE BREVET EUROPEEN**

(21) Numéro de dépôt: 88401648.6

(22) Date de dépôt: 28.06.88

(51) Int. Cl.⁴: **C 07 K 15/20**
C 08 H 1/06

(30) Priorité: 01.07.87 FR 8709297

(43) Date de publication de la demande:
04.01.89 Bulletin 89/01

(84) Etats contractants désignés:
AT BE CH DE ES GB GR IT LI LU NL SE

(71) Demandeur: DIATECH S.A.
15, rue des Grands Prés
F-92000 Nanterre (FR)

(72) Inventeur: Goldstein, Israel
47, boulevard Barbès
F-75018 Paris (FR)

(74) Mandataire: Peuscet, Jacques et al
Cabinet Peuscet 68, rue d'Hauteville
F-75010 Paris (FR)

(54) Procédé de fabrication de structures organisées de collagène, notamment d'origine humaine, et structures organisées de collagène correspondantes.

(57) L'invention concerne un procédé de fabrication de structures organisées de collagène, notamment d'origine humaine et les structures organisées de collagène correspondantes. Ces structures organisées peuvent être soit des fibres soit des membranes. Selon ce procédé, on broie dans une solution saline un échantillon de tissu animal ou humain, préalablement lavé et aseptisé, pour solubiliser le collagène contenu dans ledit échantillon. On ajoute ensuite à l'extrait obtenu, composé de fibrilles de collagène en quantité minimum et exempt de précipité collagénique non fibrillaire, un catalyseur de reconstitution des fibres de collagène. Après une étape de maturation, les structures organisées reconstituées sont séparées du milieu aqueux par centrifugation, lavées, aseptisées, conditionnées, lyophilisées, stérilisées. Parmi les structures organisées obtenues, les fibres de collagène se présentent à l'état isolé, séparées de leur tissu cellulaire d'origine, en réseau (35), prêtes à l'emploi. Elles présentent une périodicité de striations transversales (37) comprise entre 68 nm et 72 nm.

FIG. 5

**Description**

## PROCEDE DE FABRICATION DE STRUCTURES ORGANISEES DE COLLAGENE, NOTAMMENT D'ORIGINE HUMAINE, ET STRUCTURES ORGANISEES DE COLLAGENE CORRESPONDANTES.

L'invention concerne un procédé de fabrication de structures organisées de collagène, notamment d'origine humaine, et les structures organisées de collagène correspondantes.

On sait que le collagène est la protéine la plus abondante chez les mammifères. Il constitue le principal élément fibreux de la peau, des os, des tendons, des cartilages, des vaisseaux sanguins et des dents; il est présent, en plus faibles proportions, dans presque tous les organes; il se présente sous la forme de structures organisées liées au tissu cellulaire, et qui sont des fibres ou des membranes.

L'unité structurale de base d'une structure organisée de collagène est la fibrille de tropocollagène. Les fibrilles de tropocollagène s'associent soit pour former les fibres de collagène qui présentent, entre autres caractéristiques, des striations perpendiculaires à leur axe tous les 70 nm environ et une très grande résistance à la traction, soit pour former des membranes de collagène.

Les fibres et les membranes de collagène étant des constituants très importants de l'organisme humain, on a cherché à reconstituer in vitro du collagène, dont les caractéristiques physiques et biologiques soient proches de celles du collagène in vivo.

Plusieurs procédés de reconstitution de collagène existent à ce jour, qui tous ont comme point de départ un tissu animal non humain. Un de ces procédés, décrit en 1979 par M. CHVAPIL et al. dans "Medical and Surgical Applications of Collagen" (International Revue of Connective Tissue Researchs, t. 6, p. 1-55), consiste à solubiliser, en milieu acide, une partie du collagène contenu dans un tissu animal, notamment un tendon de boeuf, ayant préalablement subi un traitement enzymatique. On obtient ainsi une suspension de collagène; ce dernier est alors reconstitué soit par dialyse, soit par précipitation en milieu salin. La structure collagénique obtenue est un précipité amorphe constitué de collagène dénaturé non fibrillaire.

Cette structure n'est généralement pas directement applicable à des fins d'utilisation médicale. En effet, la matière collagénique obtenue manque de résistance à la traction en milieu humide et résiste peu à la dégradation enzymatique lors de son application sur les tissus vivants. Ce collagène doit donc préalablement subir des traitements particuliers pour pallier ces inconvénients. Une fois ces traitements effectués, le collagène peut se présenter, entre autres, sous forme de gel ou d'éponge. Il peut alors être utilisé comme agent hémostatique, comme pansement humide chirurgical ou comme pansement humide pour les brûlures.

Cependant, ce collagène dénaturé ou moléculaire bien qu'ayant été traité pour retrouver des caractéristiques physiques et biologiques proches de celles du collagène in vivo, ne donnent pas toujours satisfaction, notamment en ce qui concerne la bonne tenue mécanique des pansements humides, car la structure organisée in vivo, notamment des fibres de collagène, fait défaut dans ce collagène artificiel. De plus, la fabrication de certaines bioprothèses collagéniques nécessiterait l'utilisation de membranes de collagène en raison des propriétés mécaniques desdites membranes.

Ces arguments ont donc conduit à étudier plus particulièrement la fibrillogénèse du collagène. Des fibres reconstituées à partir de peau de lapin ont pu être mises en évidence au cours d'une expérimentation décrite en 1969 par I. GOLDSTEIN (C.R. Académie des Sciences de Paris, t. 268, p. 2446-2448), mais l'obtention de telles fibres est d'un très faible rendement, de l'ordre de 0,5% en poids (par rapport au poids de peau d'origine) si l'on part d'un extrait de peau animale.

Si ces fibres reconstituées présentent des avantages considérables par rapport aux gels de collagène dénaturé, elles restent néanmoins d'origine non humaine et sont donc susceptibles d'être rejetées par un organisme humain sur lequel elles seraient appliquées ou dans lequel elles seraient implantées.

La présente invention remédie aux inconvénients précités en proposant un procédé de reconstitution de structures organisées de collagène, notamment d'origine humaine, qui offre, par l'utilisation d'un catalyseur, un rendement d'obtention de fibres pouvant être multiplié par un facteur 5 environ par rapport à une technique n'utilisant pas ce catalyseur. Le procédé selon l'invention autorise donc une production industrielle de telles structures.

On peut notamment utiliser, comme tissu humain servant de matières premières, de la peau ou des fractions de placenta. A partir de peau, on obtiendra des fibres de collagène, et, selon la fraction de placenta utilisé, on obtiendra soit des fibres, soit des membranes de collagène.

La reconstitution de fibres de collagène à partir de peau humaine est limitée par la quantité de tissu que l'on peut prélever sur un être vivant, par exemple au cours d'une intervention de chirurgie esthétique. Bien entendu, il serait possible de mettre en oeuvre ce procédé à partir de peau de cadavre, mais, indépendamment des problèmes d'éthique, l'impact psychologique sur les patients serait très mauvais, notamment dans le cas d'une implantation de collagène pour des traitements esthétiques.

Par contre, l'obtention de placenta humain en grande quantité est possible, notamment en utilisant les organes d'ores et déjà récoltés dans les maternités et conservés au froid. Mais, dans ce cas, l'utilisation d'un procédé sans catalyseur du type de celui décrit pour la peau de lapin par GOLDSTEIN (document susmentionné) conduit à des rendements d'environ 0,02% qui sont impraticables industriellement. Au contraire, la mise en oeuvre du procédé selon l'invention, qui comporte l'utilisation d'un catalyseur de reconstitution des structures organisées, notamment des fibres permet d'obtenir un rendement de 0,1% au lieu de 0,02% ce qui permet d'envisager une production industrielle.

Les structures organisées obtenues, grâce au procédé selon l'invention, se présentent, à l'état isolé, séparées de leur tissu cellulaire d'origine. Elles présentent donc une structure analogue à celle du réseau fibrillaire collagénique in vivo. Leur origine humaine leur confère des propriétés non antigéniques et elles sont donc très bien supportées par l'homme. Pour assurer leur conservation, on peut les lyophiliser; leur lyophilisation avant stockage leur confère une très grande résistance à la dégradation biologique et leur remise en suspension dans de l'eau et/ou un tampon isotonique avant utilisation ne pose pratiquement pas de problème. Les suspensions aqueuses de fibres selon l'invention, lorsqu'elles sont utilisées sur l'homme, par exemple en injection sous-cutanée, ne produisent aucune des allergies ou des oléomes que l'on peut rencontrer lorsque l'on implante sur l'homme du collagène dermique bovin purifié (également appelé "zyderm") préalablement suspendu dans un sérum physiologique contenant 0,3% de ludocaïne et de l'huile végétale.

L'utilisation de ces structures organisées reconstituées, d'origine humaine, est multiple. On peut les utiliser, sous la forme de fibres, pour des opérations de chirurgie esthétique, ou pour fabriquer des pansements humides qui favorisent la cicatrisation des plaies ou augmentent la valeur critique de la surface de brûlure des grands brûlés. Leur utilisation est possible également pour obtenir une cicatrisation physiologique des plaies atones d'origine vasculaire ou neurologique.

De plus, la capacité de polymérisation des structures organisées avec la glutaraldéhyde permet d'envisager la fabrication de prothèses et, notamment, de valvules cardiaques. Dans un tel cas, on pourra choisir des structures organisées obtenues à partir d'une matière première correspondant à l'organe où l'on doit implanter la prothèse.

Les fibres peuvent également servir de support pour des médicaments qui n'ont besoin de se libérer que progressivement dans l'organisme.

Dans le cas d'opérations esthétiques, on peut prélever sur un patient un fragment de tissu, en extraire le collagène, et réinjecter, sur le même patient, au niveau de ses rides, les fibres reconstituées selon l'invention.

L'invention a donc pour objet un procédé de fabrication de structures organisées de collagène d'origine animale caractérisé par la succession des étapes suivantes:

a) on lave et on aseptise un tissu animal fragmenté contenant du collagène pour obtenir un échantillon exempt d'agent contaminant;

b) on solubilise au moins une partie du collagène contenu dans l'échantillon obtenu selon a) dans au moins une solution aqueuse aseptique (SA1, SA2, SA3) d'au moins un dérivé diaminé ou un sel de métal alcalin ou alcalino-terreux notamment sodium (Na), magnésium (Mg), calcium (Ca), à un pH compris entre 6,5 et 7,5 et on sépare le résidu non solubilisé pour obtenir un extrait constitué par une suspension de fibrilles de collagène, extrait que l'on ne conserve que si ladite suspension est exempte de précipité non fibrillaire et si ladite suspension comporte une quantité minimum de fibrilles permettant par exemple d'envisager la reconstitution d'une quantité de structures organisées telle que cette quantité soit industriellement et/ou rentablement acceptable.

c) on dilue la suspension obtenue selon b) avec de l'eau apyrogène et on y ajoute au moins un catalyseur de reconstitution des fibres de collagène pris dans le groupe formé par les sucres monoaminés ayant un poids moléculaire compris entre 180 et 250 et les acides uroniques ou leurs dérivés, notamment les dérivés lactone;

d) on laisse maturer le mélange selon c) pour permettre la reconstitution des structures organisées de collagène;

e) on sépare lesdites structures organisées reconstituées du milieu aqueux, on les lave et on les conditionne pour leur utilisation ultérieure.

Avantageusement, on choisit comme catalyseur, dans l'étape c), l'acide galacturonique. On choisit, de préférence, une quantité d'acide galacturonique telle que l'on obtienne un pH compris entre environ 4 et environ 5.

Dans l'étape c), on peut choisir une dilution de la suspension telle que le rapport en volume (suspension initiale/eau de dilution) soit compris entre 1/10 et 1/20, et on fait agir avantageusement le catalyseur à une température comprise entre 30°C et 50°C, de préférence à 40°C, pendant un temps compris entre 30 et 90 minutes, de préférence 60 minutes.

Avantageusement dans l'étape d), on laisse maturer ledit mélange au repos à une température comprise entre 1°C et 10°C, de préférence environ 4°C, pendant un temps compris entre 10 heures et 100 heures.

On peut séparer les structures organisées de collagène reconstituées du milieu aqueux par centrifugation, et on lave, de préférence, plusieurs fois les structures organisées de collagène séparées du milieu aqueux à l'eau apyrogène et à l'éthanol.

Avantageusement, on recycle le liquide centrifugé en y ajoutant le catalyseur de l'étape c), en le faisant maturer dans une étape analogue à l'étape d) et en extrayant les structures organisées formées dans une étape analogue à l'étape e).

On obtient, de préférence, l'échantillon exempt d'agent contaminant en fractionnant le tissu animal et en lavant au moins une fois les fragments obtenus à l'eau apyrogène et à l'éthanol et on peut laver lesdits fragments à l'éthanol sous agitation pendant un temps compris entre 1 minute et 10 heures.

Avantageusement, dans l'étape b), on solubilise au moins une partie du collagène contenu dans l'échantillon en broyant ledit échantillon dans la solution aqueuse (SA1, SA2, SA3) et en agitant le broyat ainsi obtenu; on broie, de préférence, l'échantillon pendant un temps compris entre 10 et 30 minutes à une température comprise entre 1°C et 10°C, de préférence environ 4°C, et l'on peut agiter ensuite le broyat obtenu pendant un temps compris entre 12 et 36 heures à une température comprise entre 1°C et 10°C, de préférence environ 4°C.

Avantageusement, on sépare par tamisage le résidu non solubilisé dans l'étape b) et on peut recycler le résidu séparé par tamisage dans une étape de solubilisation analogue à celle mentionnée pour l'étape b), la concentration saline de la solution aqueuse (SA1, SA2, SA3) étant augmentée d'une solubilisation à la suivante jusqu'à atteindre la concentration maximale utilisée, le nombre de recyclages étant avantageusement inférieur à 10 pour chaque solution saline mise en oeuvre et les extraits étant soumis à l'étape c). Dans une variante de l'invention, les extraits peuvent être regroupés avant d'être soumis à l'étape c).

On peut laver l'élément de tamisage avec de l'eau apyrogène, puis on ajoute avantageusement à cette eau de lavage le catalyseur de l'étape c) et enfin, après une étape de maturation analogue à celle de l'étape d), on ajoute, de préférence, ce deuxième mélange au mélange maturé de l'étape d).

Dans un premier mode de mise en oeuvre du procédé selon l'invention, on utilise, comme tissu animal, de la peau humaine. Les structures organisées obtenues sont alors des fibres de collagène. Avantageusement, dans l'étape b), on utilise, comme sel pour la solution saline de solubilisation (SA1), au moins un chlorure de métal alcalino-terreux, notamment le chlorure de calcium, à une concentration comprise entre 0,5 M et 2,5 M.

Dans un deuxième mode de mise en oeuvre du procédé, on utilise, comme tissu animal, au moins un constituant de placenta humain contenant principalement du collagène de type I et III.

Avantageusement, on effectue l'étape de solubilisation selon b) successivement avec au moins deux solutions différentes (SA1, SA2, SA3), la première (SA1) contenant au moins un sel de métal alcalin, notamment le chlorure de sodium, alors que la seconde (SA2) contient au moins un sel de métal alcalino-terreux, notamment le chlorure de calcium.

Avantageusement, le résidu obtenu après la dernière solubilisation dans la première solution saline (SA1) est lavé au moins une fois à l'eau apyrogène puis est soumis à la deuxième solution saline (SA2) dans laquelle, par recyclage des résidus non solubilisés, on peut réaliser plusieurs solubilisations successives, tous les extraits obtenus par une solubilisation étant soumis à la catalyse de l'étape c). Les structures organisées obtenues sont alors des fibres de collagène. Dans une variante de l'invention, les extraits peuvent être regroupés avant d'être soumis à la catalyse de l'étape c). Afin d'extraire le collagène de type IV, V ou VI qui serait en outre éventuellement contenu dans le constituant de placenta humain de départ, le résidu obtenu après la dernière solubilisation dans la deuxième solution saline (SA2) peut être lavé au moins une fois à l'eau apyrogène et peut être soumis à une troisième solution (SA3), contenant au moins un dérivé diaminé, dans laquelle par recyclage des résidus non solubilisés, on réalise plusieurs solubilisations successives, tous les extraits obtenus par la solubilisation dans la solution saline (SA3) étant soumis à la catalyse de l'étape c). Les structures organisées obtenues sont alors des membranes de collagènes.

Dans un troisième mode de mise en oeuvre du procédé selon l'invention , on choisit comme constituant de placenta humain, un constituant contenant principalement du collagène de type IV, V, ou VI. Les structures organisées obtenues sont alors des membranes de collagène. Avantageusement, on effectue l'étape de solubilisation selon b) avec au moins une solution (SA3) contenant au moins un dérivé diaminé. Cependant, afin de purifier le constituant placentaire de départ du collagène de type I et III qu'il pourrait éventuellement contenir, on peut utiliser dans l'étape de solubilisation selon b), préalablement à la solution (SA3), successivement deux solutions salines différentes (SA1, SA2), la première (SA1) contenant au moins un sel de métal alcalin, notamment le chlorure de sodium, alors que la seconde (SA2) contient au moins un sel de métal alcalino-terreux, notamment le chlorure de calcium.

Dans un quatrième mode de mise en oeuvre du procédé, on utilise, comme tissu animal, un placenta humain entier. Avantageusement, on effectue l'étape de solubilisation selon b) successivement avec au moins trois solutions différentes (SA3, SA1, SA2), la première (SA3) contenant au moins un dérivé diaminé, afin d'éliminer la majeure partie du collagène de type IV, V ou VI contenu dans le placenta de départ, la deuxième (SA1) contenant au moins un sel de métal alcalin et la troisième (SA2) contenant au moins un sel de métal alcalino-terreux.

De préférence, le résidu obtenu après la deuxième solubilisation dans la première solution (SA3) est lavé au moins une fois à l'eau apyrogène, puis est avantageusement soumis à la deuxième solution (SA1), dans laquelle, par recyclage des résidus non solubilisés, on réalise plusieurs solubilisations successives ; le résidu obtenu après la dernière solubilisation dans la deuxième solution (SA1) est de préférence lavé au moins une fois à l'eau apyrogène, puis est avantageusement soumis à la troisième solution (SA2) dans laquelle, par recyclage des résidus non solubilisés, on réalise plusieurs solubilisations successives, la catalyse de l'étape c) étant appliquée, de préférence, au moins à tous les extraits obtenus par les solubilisations dans les solutions (SA1, SA2). Les structures organisées de collagène obtenues sont alors des fibres de collagène.

Afin d'extraire le collagène de type IV, V ou VI qui serait éventuellement encore présent dans le résidu obtenu après la dernière solubilisation dans la troisième solution (SA2), ledit résidu est, de préférence, lavé au moins une fois à l'eau apyrogène, puis est avantageusement soumis à une quatrième solution (SA3) contenant au moins un dérivé diaminé, dans laquelle, par recyclage des résidus non solubilisés, on réalise plusieurs solubilisations successives, tous les extraits obtenus par les solubilisations dans la quatrième solution (SA3) étant soumis à la catalyse de l'étape c). Les structures organisées de collagène obtenues sont alors des membranes de collagène.

Avantageusement, on choisit comme sel de métal alcalino-terreux le chlorure de calcium à une concentration comprise entre 0,5 M environ et 1,5 M environ, et on peut choisir comme sel de métal alcalin le chlorure de sodium à une concentration comprise entre 0,5 M environ et 2 M environ. On choisit, de préférence, le dérivé diaminé dans le groupe formé par la guanidine, l'urée et leurs sels et on peut utiliser une concentration pour la solution de solubilisation (SA3) comprise entre environ 2 M et environ 6 M.

On peut alors conditionner les structures organisées obtenues par lyophilisation puis par stérilisation, notamment par irradiation électronique, par exemple au moyen d'un accélérateur d'électrons avec une dose d'irradiation de 2,6 Mrad.

L'invention concerne également des fibres de collagène caractérisées par le fait que:

- elles sont d'origine humaine;
- elles sont à l'état isolé, séparées de leur tissu cellulaire d'origine, constituées en réseau et prêtes à l'emploi;
- elles ont une longueur comprise entre 0,1 mm et 4 mm;
- elles ont une dimension transversale comprise entre 100 nm et 500 nm;
- elles ont une périodicité de striations transversales comprise entre 68 nm et 72 nm.

L'invention concerne enfin des structures organisées de collagène caractérisées par le fait qu'elles sont obtenues par le procédé décrit ci-avant.

Afin de mieux faire comprendre l'objet de l'invention, on va en décrire ci-après, à titre purement illustratif et non limitatif, trois modes de mise en oeuvre représentés sur le dessin ci-annexé.

Sur ce dessin:
- la figure 1 représente un organigramme d'un premier mode de mise en oeuvre du procédé selon l'invention appliqué à de la peau humaine fragmentée;
- la figure 2 est un organigramme d'un deuxième mode de mise en oeuvre du procédé selon l'invention appliqué à une fraction de placenta humain contenant principalement du collagène de type I et III;
- la figure 3 est un organigramme d'un troisième mode de mise en oeuvre du procédé selon l'invention appliqué à une fraction de placenta humain contenant principalement du collagène de type IV, V ou VI;
- la figure 4 est un organigramme d'un quatrième mode de mise en oeuvre du procédé selon l'invention appliqué à un placenta humain entier, et,
- la figure 5 représente une vue au microscope électronique d'une partie d'un réseau de fibres de collagène obtenues par le procédé selon l'invention.

Dans les quatre modes de mise en oeuvre du procédé, toutes les manipulations devront être effectuées dans des conditions au moins de stérilité chirurgicale et à la température de 4°C.

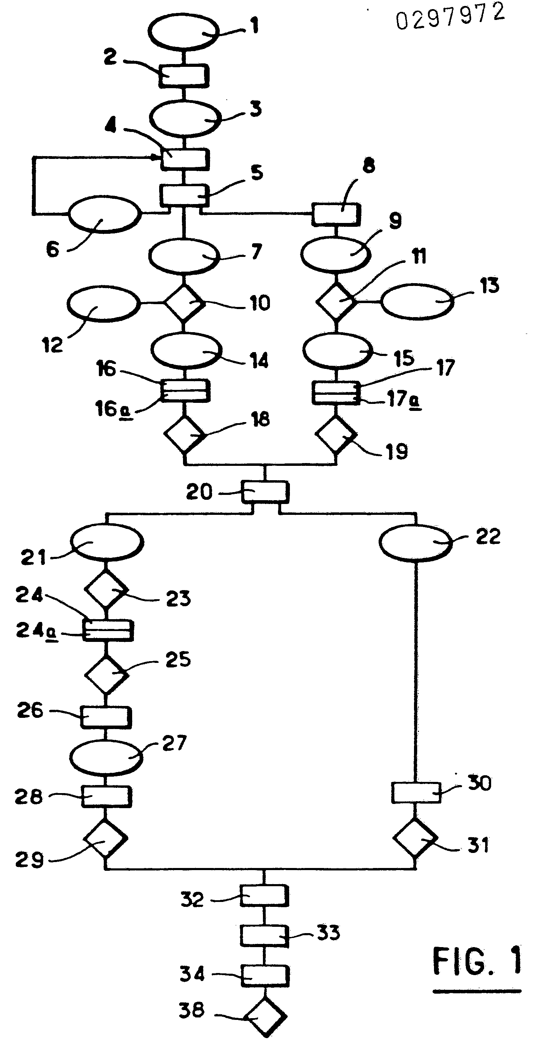

Dans un premier mode de mise en oeuvre, on applique le procédé, dont l'organigramme est représenté sur la figure 1, à de la peau humain dégraissée, provenant par exemple de chutes d'opérations esthétiques. Dans ce cas, comme précisé ci-avant, les structures organisées obtenues sont des fibres de collagène.

Ces chutes de peau sont fragmentées et coupées en lanières fines de façon à obtenir des fragments (opération 1).

Ces fragments de peau sont ensuite soumis à une étape de lavage et d'aseptisation 2. Cette étape consiste à laver au moins cinq fois dans l'eau distillée, froide, stérile, apyrogène, les fragments issus de l'opération 1, de façon à les débarrasser du sang éventuel provenant de la peau d'origine et à les aseptiser en les traitant avec de l'éthanol pur à 70% en poids. Ce traitement alcoolique consiste à plonger les fragments dans l'éthanol et à les agiter énergiquement pendant 2 heures à une température de 4°C. Cette température contribue à éviter une éventuelle surinfection et à empêcher l'activité enzymatique. Cette utilisation de l'alcool est nécessaire, d'une part, pour désinfecter les fragments de peau, en tuant les germes banaux, en détruisant les virus, notamment les virus encapsulés du type HIV (SIDA) et hépatite B, et, d'autre part, pour inactiver toutes les enzymes protéolytiques, qui dégradent les fibres de collagène. Une fois ce traitement à base d'éthanol terminé, les fragments de peau aseptisés sont relavés avec de l'eau distillée, froide, stérile, apyrogène, afin d'éliminer toute trace d'alcool. On obtient alors un échantillon 3 exempt de tout agent contaminant.

Cet échantillon 3 va ensuite être soumis à une étape de solubilisation 4 du collagène et des autres macromolécules qu'il contient. La peau contenant principalement du collagène de type I, il est nécessaire pour solubiliser ce collagène d'utiliser une solution aqueuse contenant un sel de métal alcalino-terreux.

On prépare donc une solution aqueuse (SA1) molaire de chlorure de calcium, tamponnée à pH 7,3 avec un tampon (citrate de sodium/acide citrique) à concentration finale de 0,05 M. On plonge alors l'échantillon aseptisé 3 dans cette solution aqueuse (SA1) et l'on choisit une quantité de solution aqueuse, telle que l'on ait un rapport en volume (solution aqueuse/échantillon 3) égale à 2/1. On broie ensuite l'échantillon 3 dans cette solution aqueuse (SA1) pendant environ 20 minutes à l'aide d'un broyeur à couteaux classique, à une température de 4°C. Pour bien imbiber le broyat obtenu de la solution (SA1), on agite ensuite énergiquement ce dernier pendant 24 heures à cette même température.

A la suite de cette solubilisation, une partie seulement du collagène contenu dans l'échantillon 3 a été solubilisée. Le mélange broyat-solution (SA1) subit alors une phase de tamisage 5, successivement à l'aide de deux tamis métalliques, inoxydables et stériles, le premier ayant un réseau de 1 mm, et l'autre un réseau de 0,5 mm. On obtient alors, d'une part, un résidu non solubilisé 6 et un extrait 7 constitué par une suspension de fibrilles de collagène.

Cet extrait 7 est examiné au microscope électronique dans une phase de contrôle 10. Ce contrôle a pour but de s'assurer que l'on obtient effectivement une suspension de fibrilles de collagène bien formées et bien distinctes et sur lesquelles ne sont pas accrochés des précipités non fibrillaires de collagène dénaturé. Un extrait 12, contenant de tels précipités non fibrillaires, serait alors automatiquement rejeté.

Si la suspension a franchi avec succès ce contrôle au microscope électronique, elle est stockée à 4°C avant l'étape ultérieure.

Dans le même temps, le résidu 6 est recyclé une première fois dans l'étape de solubilisation 4, dans laquelle la concentration de chlorure de sodium a été augmentée pour atteindre la valeur de 1,5 M. La valeur du pH est toujours maintenue à 7,3 et le rapport en volume (solution/résidu) est toujours égal à 2/1. Généralement, dans le cas d'un échantillon 3 provenant de peau humaine, d'une part, les deux premiers extraits ne sont pas exempts de précipités non fibrillaires (il est donc nécessaire de les rejeter) et d'autre part, on solubilise la majeure partie du collagène contenu dans l'échantillon 3 en effectuant six recyclages du résidu 6 dans l'étape de solubilisation 4. Lors de ces recyclages, du deuxième au sixième, la concentration de chlorure de calcium dans la solution saline (SA1) est prise égale à 2M. Cependant, le nombre de ces recyclages est fonction du degré de solubilisation des fibres, qui est d'autant plus faible que la peau d'origine utilisée est vieille. Le résidu 6 non solubilisé à l'issue de recyclage complet peut être traité chimiquement par des procédés de l'état de la technique pour produire un gel de collagène dénaturé non fibrillaire.

Les extraits sélectionnés après le contrôle 10, généralement les cinq derniers, sont mélangés et la suspension résultante 14 est soumise à une étape de catalyse 16 et de maturation 16a. Cette étape catalysante 16 consiste à diluer la suspension 14 obtenue avec de l'eau apyrogène et à y ajouter un catalyseur de reconstitution des fibres de collagène. On choisit comme catalyseur l'acide galacturonique, que l'on ajoute à raison de 0,1 mg/ml de suspension 14. On choisit, d'autre part, une dilution de la suspension 14, telle que l'on obtienne un pH de ladite suspension égal à 4,5. Généralement, le rapport en volume (suspension initiale/eau de dilution) est compris entre 1/10 et 1/20.

L'acide galacturonique a été choisi car, parmi les constituants biochimiques normaux du tissu conjonctif, c'est celui qui fournit le meilleur rendement d'obtention de fibres de collagène. Cependant, on aurait pu choisir des sucres monoaminés, ayant un poids moléculaire compris entre 180 et 250, ainsi que les acides uroniques en général, ou leurs dérivés lactone. Cependant, dans le cas où l'on choisit un dérivé lactone, il faut ajuster le pH aux alentours de 5. L'ajustement de la valeur du pH et la quantité d'acide galacturonique ajoutée sont très importants car, si l'on augmente la quantité d'acide galacturonique, ce qui revient donc à abaisser le pH, on augmente la précipitation du collagène, non pas sous forme de fibres, mais sous forme de précipités amorphes de collagène dénaturé.

Une fois l'acide galacturonique ajouté à la suspension, on incube le mélange ainsi obtenu pendant 1 heure à 40°C. Cette incubation a pour but de faciliter la croissance des fibres de collagène, mais il doit être limité dans le temps pour éviter les développements bactériens et microbiens éventuels.

Une fois le mélange chauffé, on laisse maturer celui-ci au repos dans l'étape 16a pendant un temps compris entre 24 heures et 72 heures à une température de 4°C, temps pendant lequel le processus de reconstitution des fibres de collagène se déroule.

Parallèlement aux traitements successifs effectués sur les extraits 7, les deux tamis utilisés dans la phase de tamisage 5 sont lavés à chaque cycle dans une phase 8 avec de l'eau distillée, stérile, apyrogène, afin de détacher le collagène qui s'est spontanément formé sur les réseaux des tamis. Chaque eau de lavage 9 est alors récupérée et soumise à un contrôle 11 identique au contrôle 10 de l'extrait 7, afin de détecter la nature du collagène en suspension dans l'eau de lavage. Une eau de lavage, contenant des fibrilles de collagène exempte de précipité non fibrillaire, est conservée, tandis qu'une eau de lavage 13, contenant des précipités collagéniques dénaturés, est rejetée. Toutes les eaux de lavage, généralement les cinq dernières, qui ont été sélectionnées, sont mélangées et ce deuxième mélange 15 est soumis à une étape de catalyse 17 et de maturation 17a identique à l'étape de catalyse 16 et de maturation 16a de la suspension 14.

Le mélange, contenant les fibres de collagène reconstituées provenant de la suspension 14 ainsi que le mélange contenant des fibres de collagène reconstituées provenant du mélange 15 des eaux de lavage des tamis, sont contrôlés séparément au microscope électronique dans des phases de contrôle 18 et 19 identiques aux phases de contrôle 10 et 11, et, si la qualité des fibres reconstituées est correcte, les deux mélanges sont ajoutés l'un à l'autre et soumis à une phase de séparation 20 des fibres de collagène du milieu aqueux.

Cette phase de séparation 20 s'effectue à 4°C en centrifugeant le mélange résultant à 4.000 tours/mn pendant une demi-heure, dans des conditions de stérilité. De cette phase de centrifugation 20, on retire, d'une part, des fibres de collagène reconstituées 22 et, d'autre part, un liquide centrifugé 21.

Le liquide centrifugé 21, qui contient des fibrilles de collagène, non encore reconstituées en fibres, ou des fibres de collagène non totalement formées, est soumis, après une phase de contrôle 23 identique à la phase de contrôle 10, à une étape de catalyse 24 et de maturation 24a identique aux étapes 16 et 16a. La qualité des fibres reconstituées lors de ces étapes 24 et 24a est contrôlée au microscope électronique dans une phase de contrôle 25 et lesdites fibres sont séparées du milieu aqueux par une centrifugation 26 analogue à la centrifugation 20.

Les fibres de collagène 27, issues du liquide centrifugé 21, et les fibres de collagène 22, issues de la phase de centrifugation 20, sont longuement lavées et aseptisées dans des phases de lavage 28 et 30 analogues à la phase de lavage 2, afin d'éliminer les sels et agents extérieurs utilisés lors des traitements précédents. Ces fibres, une fois lavées et débarrassées de toute trace d'alcool, sont soumises à deux phases de contrôle identiques 29 et 31, comportant un examen au microscope électronique et des tests immunologiques.

Ces tests utilisent des techniques immunoenzymologiques pour la recherche des protéines sériques solubles et la recherche des protéines de structure autre que le collagène. La première de ces recherches s'effectue à l'aide des globulines de lapin anti-sérum humain marquées à la péroxydase (immunopéroxydase directe) ; la seconde recherche s'effectue par une technique similaire (immunopéroxydase indirecte).

Les fibres, conformes aux critères de sélection, sont ensuite mélangées et homogénéisées dans une phase de conditionnement 32 dans laquelle elles sont stockées dans des flacons de verre neutre de 50 ml.

Les flacons sont ensuite lyophilisés dans l'étape 33, ce qui permet aux fibres de conserver leur stabilité et d'acquérir une grande résistance à la dégradation biologique.

Les flacons sont enfin stérilisés, dans l'étape 34, par une irradiation électronique au moyen d'un accélérateur d'électrons avec une dose d'irradiation de 2,6 Mrad, ce qui n'altère pas les propriétés biologiques des fibres.

L'étape 38 est un contrôle final de la préparation obtenue. Ce contrôle consiste en un examen morphologique au microscope électronique, et en une série de tests biochimiques, biologiques, bactériologiques, afin de préciser la nature exacte de la préparation, son uniformité, sa stérilité, sa stabilité.

Les flacons de 50 ml sont ensuite conservés à 4°C jusqu'à leur utilisation ultérieure qui nécessitera, 24 heures auparavant, la réhydratation des fibres par leur resuspension dans 10 ml d'eau physiologique stérile, tamponnée à pH égal à 7,3 avec un tampon phosphate de concentration égale à 0,1 M.

Le rendement d'un tel procédé est de l'ordre de 5% en poids (par rapport au poids des fragments de peau de départ). Il serait de l'ordre de 0,5% en poids si l'on n'utilisait pas de catalyseur de reconstitution des fibres.

La reconstitution des fibres de collagène à partir de peau humaine est évidemment limitée par la quantité de tissu que l'on peut prélever sur un être vivant. C'est pourquoi, selon l'invention, on utilise aussi, comme tissu humain servant de matière première, des fractions de placenta contenant principalement du collagène de type I et III. Le placenta humain peut être récolté en grande quantité, notamment, dans les maternités, et être conservé au froid à -20°C. Le placenta contient en grandes quantités des protéines d'origine sanguine, des enzymes (telles que la protéase, la collagénase, l'élastase), des hormones (telles que la prolactine, l'hormone gonadotrope, la folliculine), des facteurs de croissance et enfin, du collagène. Ce dernier est situé principalement dans le cordon ombilical, dans le placenta chorial, dans les parois de vaisseaux, dans la membrane amniotique et dans le squelette conjonctif du placenta. Chacun des éléments précités contient des types différents de collagène. Ainsi, on trouve du collagène de type I et III dans les vaisseaux et le cordon ombilical, du collagène de type V et VI dans la membrane amniotique, et enfin, du collagène de type IV dans le placenta chorial. Le collagène de type I et III donne des fibres, tandis que le collagène de type IV, V ou VI génère des membranes.

Le placenta humain récolté dans les maternités et gardé au froid n'est pas directement exploitable pour le procédé selon l'invention. Il est nécessaire, dans un premier temps, de broyer ce placenta et de le traiter avec une solution de chlorure de sodium à 0,9% et d'éthanol à 8%, puis de le sécher et de le recongeler à -20°C. On obtient alors un stroma placentaire sur lequel on peut appliquer le procédé selon l'invention. Cependant, compte tenu de la complexité de la constitution du placenta et de la pluralité des types de collagène contenus dans cet organe, l'application du procédé de reconstitution de structures organisées à un stroma placentaire entier est, si l'on ne prend pas des mesures particulières, vouée à l'échec et ne donnera ni des fibres ni des membranes mais des précipités amorphes de collagène. Une de ces mesures consiste à extraire de ce stroma placentaire des fractions contenant, par exemple, soit principalement du collagène de type I et III, soit principalement du collagène de type IV, V ou VI, suivant la nature des structures organisées que l'on souhaite obtenir. Une autre mesure consiste à appliquer un traitement biochimique particulier à un stroma placentaire entier afin de se ramener au cas de fractions placentaires contenant principalement du collagène de type I et III. On obtiendra alors essentiellement des fibres de collagène.

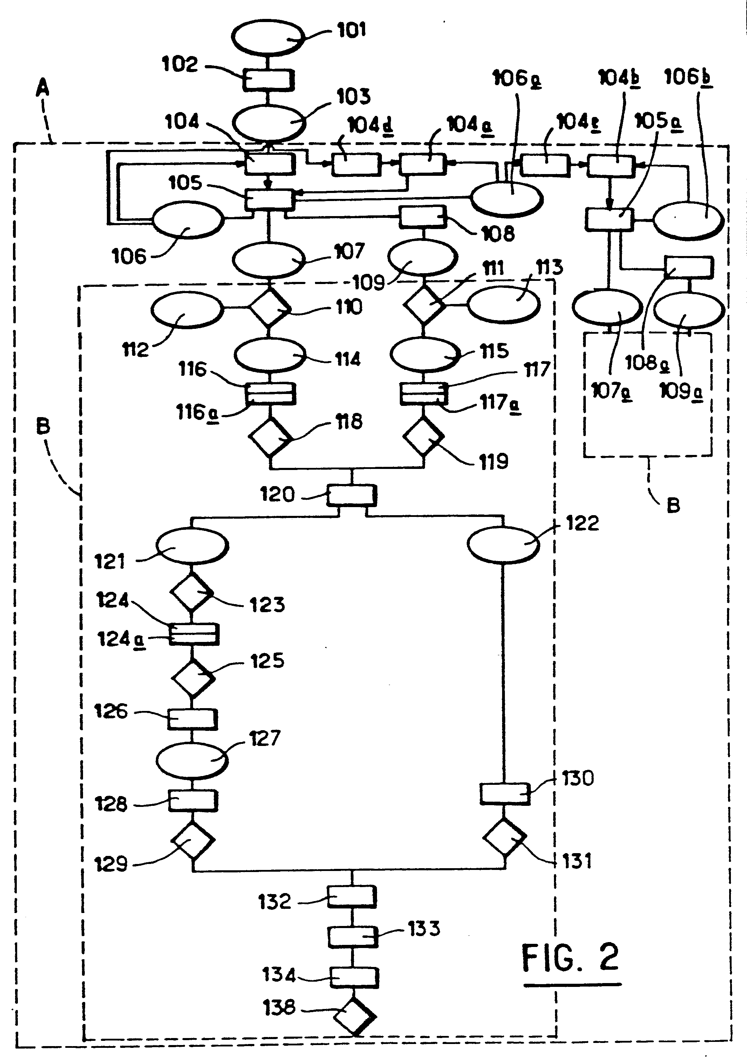

Si l'on se réfère maintenant à la figure 2, on voit que l'on a représenté un organigramme d'un second mode de mise en oeuvre du procédé selon l'invention, dont les éléments analogues à ceux de l'organigramme du premier mode de mise en oeuvre ont été repérés sur cette figure par des chiffres de référence supérieurs de 100. On ne décrira ici que les différences entre ces deux modes de mise en oeuvre.

Ce second mode de mise en oeuvre s'applique à une fraction de stroma placentaire contenant principalement du collagène de type I et III. La fraction de stroma placentaire 101, ayant été lavée et aseptisée dans une phase de lavage 102, fournit donc un échantillon 103 prêt à subir une phase de solubilisation. Le collagène de type III contenu dans l'échantillon 103 est composé de fibres plus jeunes et plus facilement solubilisables que le collagène de type I. C'est pourquoi, se l'on utilisait dans l'étape de solubilisation 104 le même sel, à savoir le chlorure de calcium, que l'on avait utilisé dans l'étape de solubilisation 4 pour la peau, on obtiendrait un précipité non fibrillaire de collagène dénaturé. Dans la suite du texte, la série de traitements allant de l'étape de solubilisation 104 jusqu'au contrôle final 138 (analogue au contrôle final 38) est désignée par la lettre A.

On prépare donc, dans un premier temps, dans cette étape de solubilisation 104, une première solution aqueuse (SA1) contenant du chlorure de sodium à une concentration égale à 0,5 M. Cette solution est tamponnée à pH égal 7,3 avec le même tampon que celui utilisé dans la phase de solubilisation 4, mais le rapport en volume (solution (SA1)/échantillon) est égal à 5/1. L'échantillon 103 est ensuite broyé dans cette solution saline (SA1) comme au cours l'étape 4. Après une étape de tamisage 105, le résidu non solubilisé 106 obtenu est recyclé dans l'étape de solubilisation 104 en augmentant d'un cycle au suivant la concentration de chlorure de sodium, le nombre de cycles étant limité à sept. Ainsi, on utilisera une concentration égale à 1 M pour le deuxième cycle, égale à 1,5 M pour le troisième et le quatrième cycle et égale à 2 M pour le cinquième, le sixième et le septième cycle.

Le résidu 106, obtenu après la dernière solubilisation dans la première solution saline (SA1) est longuement lavé à l'eau apyrogène dans une étape de lavage 104d afin d'éliminer cette première solution saline puis est soumis à une deuxième étape de solubilisation 104a, qui utilise une deuxième solution saline (SA2). Cette deuxième solution (SA2) diffère de la solution (SA1) par la nature du sel utilisé. En effet, on peut supposer, après l'étape de solubilisation 104, que la majeure partie du collagène de type III a été solubilisée. Il reste donc à solubiliser le collagène de type I et l'on utilise alors, comme sel pour cette deuxième solution saline (SA2), le chlorure de calcium à une concentration allant de 0,5 M pour le premier cycle jusqu'à 1,0 M pour le dernier cycle. On effectue également, dans cette deuxième étape de solubilisation 104a, six recyclages du résidu 106a ; on choisit, pour le deuxième cycle, une concentration de chlorure de calcium égale à 0,5 M, ainsi que pour les troisième et quatrième cycles, et une concentration de chlorure de calcium égale à 1 M, pour les cinquième, sixième et septième cycles.

On peut poursuivre la phase de solubilisation 104a en effectuant deux recyclages supplémentaires du résidu 106a dans la solution saline (SA2) molaire dans laquelle on a ajouté un réducteur non toxique , le dithiothréitrole, à une concentration finale de 2 mM. La présence du dithiothréitrole dans la solution saline (SA2) favorise l'extraction des fibres qui seraient encore présentes dans le résidu 106a. A l'issue de ces deux phases de solubilisation successives 104 et 104a, on obtient seize extraits 107 et seize eaux de lavage des tamis 109.

Ces extraits 107 et ces eaux de lavage 109 sont alors soumis à des contrôles 110 et 111, afin de vérifier la qualité des suspensions de fibrilles obtenues et d'éliminer les extraits et les eaux de lavage qui contiennent des suspensions de fibrilles de collagène accompagnées de précipités de matières collagéniques dénaturées. Les extraits et les eaux de lavage des tamis sélectionnés après ces phases de contrôle sont mélangés pour former un premier mélange 114 d'extraits et un deuxième mélange d'eaux de lavage 115. Généralement, comme dans le cas de la peau, les deux premiers extraits 107 et les deux premières eaux de lavage des tamis 109 sont rejetés. Les deux mélanges 114 et 115 subissent alors respectivement une étape de catalyse (116, 117) et de maturation (116a et 117a) analogues aux étapes 16, 17 et 16a, 17a utilisées pour la peau humaine et les traitements suivants sont identiques à ceux utilisés pour le premier mode de mise en oeuvre du procédé représenté figure 1. Dans la suite du texte, la série de traitements allant des phases de contrôle 110 et 111 des extraits et des eaux de lavage des tamis, jusqu'au contrôle final 138 est désignée par la lettre B.

On peut supposer après cette série de traitements que l'on a reconstitué pratiquement la quasi totalité des fibres de collagène de type I et III , mais comme cette fraction de stroma provenait de résidus placentaires, il est possible que le dernier résidu non solubilisé 106a contienne également du collagène de type IV, V, ou VI. Il est alors intéressant d'en extraire des membranes de collagène. C'est pourquoi parallèlement aux traitements effectués sur les seize extraits 107 et les seize eaux de lavage des tamis 109, on peut effectuer sur ce résidu 106a obtenu après le seizième recyclage une série de traitements décrits ci-après.

Le dernier résidu 106a obtenu est lavé longuement à l'eau apyrogène dans une phase de lavage 104e, analogue à la phase de lavage 104d, de façon à éliminer la solution saline (SA2) et le dithiothréitrole , puis est recyclé dans une étape de solubilisation 104b, analogue à l'étape 104a, mais dans laquelle la solution saline (SA2) est remplacée par une solution (SA3). Dans cette dernière solution (SA3), on n'utilise plus un sel de chlorure métallique mais un dérivé diaminé. Le dérivé diaminé choisi est le chlorhydrate de guanidine. La solution (SA3) est également tamponnée à pH égal à 7,3 et on y ajoute également du dithiothreitrole, à concentration finale de 2 mM. Le rapport en volume (Solution (SA3)/résidu 106a) est égal à 5/1. Le résidu 106a lavé est broyé une première fois, dans l'étape 104b, dans la solution (SA3) dans laquelle la concentration de chlorhydrate de guanidine est égale à 4 M puis après une phase de tamisage 105a, analogue à la phase 105, le résidu 106b obtenu est recyclé une fois dans cette étape 104b, la concentration de chlorhydrate de guanidine étant égale à 6 M. A l'issue de cette phase de solubilisation 104b, on obtient deux extraits 107a et deux eaux de lavage de tamis 109a (lesquels tamis ont été lavés dans une phase 108a analogue à la phase 108) auxquels on fait subir des traitements analogues à la série de traitements B pour les seize extraits 107 et 109 précédents. On obtiendra alors des membranes reconstituées de collagène 122 et 127.

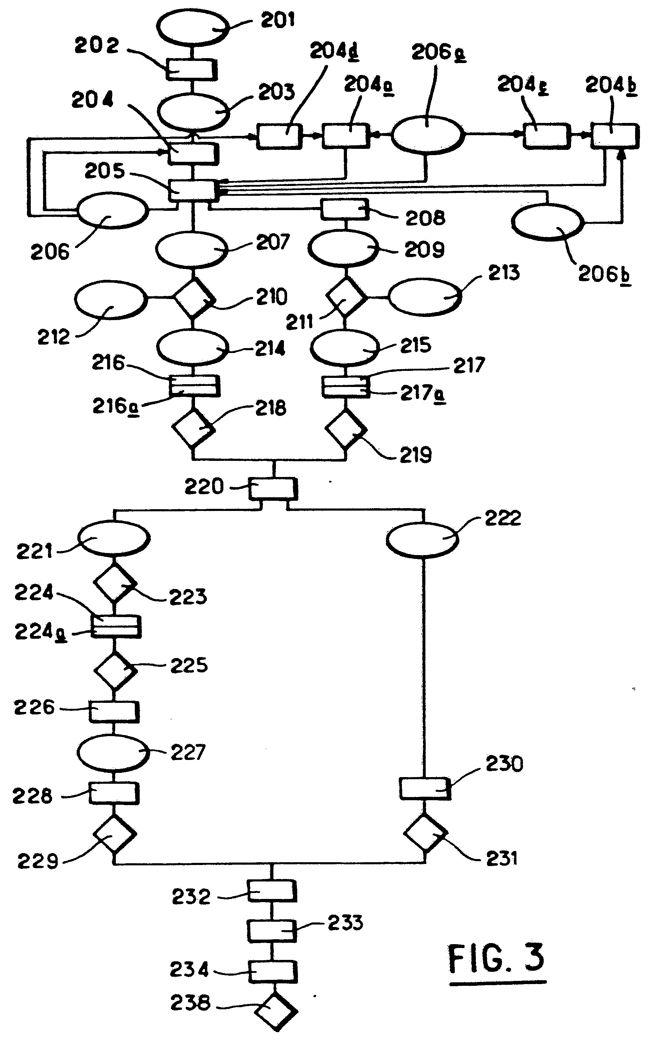

Si l'on se réfère maintenant à la figure 3, on voit que l'on a représenté un organigramme d'un troisième mode de mise en oeuvre du procédé selon l'invention, dont les éléments analogues à ceux de l'organigramme du deuxième mode de mise en oeuvre ont été repérés sur cette figure par des chiffres de référence supérieurs de 100. On ne décrira ici que les différences entre ces deux modes de mise en oeuvre.

Ce troisième mode de mise en oeuvre du procédé s'applique à une fraction 201 de stroma placentaire contenant principalement du collagène de type IV, V ou VI. Cependant cette fraction 201 peut contenir, pour les mêmes raisons que celles exposées ci-dessus, du collagène de type I et III en faible quantité. L'échantillon 203, obtenu à partir de cette fraction 201, devra donc être "purifié" de ce collagène de type I et III afin d'en extraire, à l'issue du procédé, uniquement des membranes de collagène de type IV, V ou VI.

C'est pourquoi on va d'abord soumettre l'échantillon 203 à une série de solubilisation 204, 204a, analogues aux phases 104 et 104a, afin d'extraire le collagène de type I et III. A l'issue de ces phases de solubilisation, on obtient seize extraits 207 et seize eaux de lavage des tamis 209. Les quatorze extraits et eaux de lavage des tamis provenant des solubilisations en présence de chlorure de sodium et de chlorure de calcium, ainsi que la première solubilisation en présence du chlorure de calcium additionné de dithiothréitrole sont rejetés car ils ne contiennent que des fibrilles de collagène de type I et III en quantité infime ne permettant pas une reconstitution, rentablement acceptable à l'échelle industrielle, des fibres de collagène à partir de ces fibrilles.

On peut supposer qu'après la première solubilisation en présence de chlorure de calcium additionné de dithiothréitrole, l'échantillon 203 de départ a été pratiquement totalement "purifié" du collagène du type I et III, et que l'on peut maintenant extraire le collagène de type IV, V ou VI.

On conserve donc l'extrait 207 et l'eau de lavage des tamis 209 provenant de la dernière solubilisation en présence du chlorure de calcium additionné de dithiothreitrole et l'on lave longuement le dernier résidu non solubilisé 206a dans une phase de lavage 204e, analogue à la phase 104e, puis on soumet ce résidu lavé à une phase de solubilisation 204b, analogue à la phase 104b, de façon à obtenir à l'issue de cette étape 204b, deux extraits supplémentaires 207 et deux eaux de lavage supplémentaires des tamis 209.

Ces trois extraits 207 et eaux de lavage des tamis 209 sont alors contrôlés et subissent des traitements analogues à ceux du deuxième mode de mise en oeuvre du procédé.

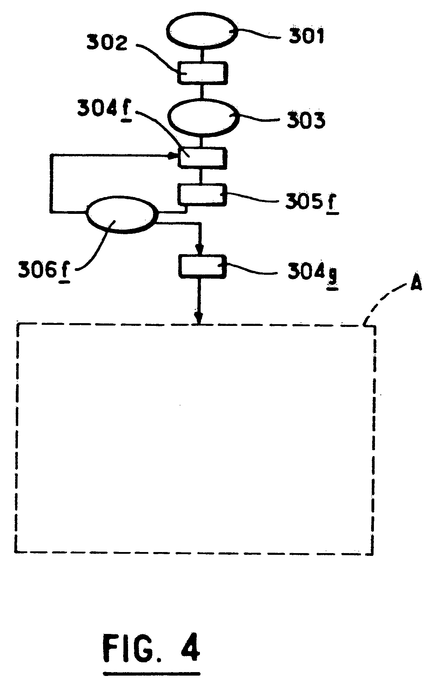

Si l'on se réfère maintenant à la figure 4, on voit que l'on a représenté un organigramme d'un quatrième mode de mise en oeuvre du procédé selon l'invention, dont les éléments analogues à ceux de l'organigramme du deuxième mode de mise en oeuvre ont été repérés sur cette figure par des chiffres de référence supérieurs de 200 à ceux utilisés pour ledit deuxième mode de mise en oeuvre. On ne décrira ici que les différences entre ces deux modes de mise en oeuvre.

Ce quatrième mode de mise en oeuvre s'applique à un stroma placentaire entier et permet par un traitement biochimique d'éliminer la majeure partie du collagène de type IV, V ou VI contenu dans le stroma afin d'obtenir un stroma placentaire contenant principalement du collagène de type I et III. Ce traitement biochimique évite toute manipulation ayant pour but de sélectionner, avant mise en oeuvre du procédé, des constituants spécifiques de stroma placentaire.

Le stroma placentaire fragmenté 301 ayant été lavé et aseptisé dans une phase de lavage 302, fournit donc un échantillon 303 prêt à subir le traitement biochimique évoqué ci-dessus. Ce traitement consiste à effectuer une phase de solubilisation 304f identique à la phase de solubilisation 104b du deuxième mode de mise en oeuvre ; on peut toutefois utiliser comme dérivé diaminé, dans la solution aqueuse (SA3) précédemment définie, l'urée à la place de la guanidine.

L'échantillon 303 est alors broyé une première fois, dans l'étape 304f, dans une solution (SA3) du type précédemment défini, dans laquelle la concentration de chlorhydrate de guanidine est égale à 4 M ; puis, après une phase de tamisage 305f analogue à la phase 105a, le résidu 306f obtenu est recyclé une fois dans cette étape 304f, la concentration de chlorhydrate de guanidine étant égale à 6 M.

A l'issue de cette phase de solubilisation 304f, on peut supposer que la majeure partie du collagène de type IV, V ou VI a été extrait de l'échantillon 303 qui devient alors analogue à un échantillon 103 contenant principalement du collagène de type I et III. Les extraits et eaux de lavage des tamis issus des phases 304f et 305f sont éliminés et le résidu 306f, obtenu après la phase 304f, est longuement lavé à l'eau apyrogène dans une étape de lavage 304g, identique à l'étape 104d, avant de subir la série de traitements A dudit deuxième mode de mise en oeuvre. On obtiendra alors principalement des fibres de collagène de type I et III et, éventuellement, comme indiqué dans le deuxième mode de mise en oeuvre, des membranes de collagène.

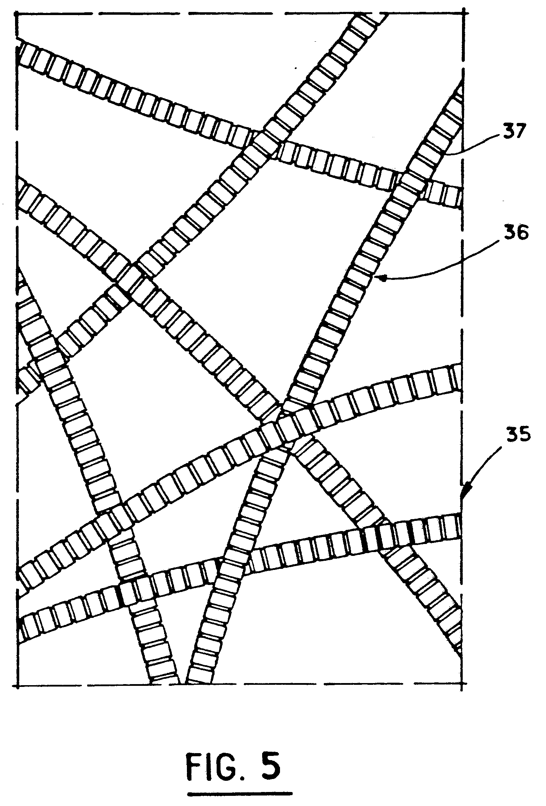

Parmi les structures organisées obtenues par le procédé selon l'invention, des fibres sont représentées sur la figure 5. Elles se présentent à l'état isolé, séparées de leur tissu cellulaire d'origine, constituées en un réseau 35. Le réseau 35 est non orienté, car l'orientation des fibres de collagène se fait dans l'organisme sur lequel elles sont appliquées ou dans lequel elles sont implantées, sous l'influence des lignes de force ou de pression exercée sur le tissu conjonctif en formation.

Ces fibres 36 ont une longueur comprise entre 0,1 mm et 4 mm, et elles ont une dimension transversale comprise entre 100 nm et 500 nm. Elles sont constituées d'un assemblage de deux complexes protéiques de structure: le collagène constitue les fibres longitudinales, et les glycoprotéines constituent les striations transversales 37 et la gaine des fibres. La périodicité de ces striations transversales est comprise entre 68 nm et 72 nm.

L'absence d'utilisation de substances toxiques au cours de la fabrication, ainsi que l'origine humaine des fibres, évitent tout risque de toxicité et de réaction allergique.

Les fibres ainsi lyophilisées sont prêtes à l'emploi et peuvent notamment être utilisées directement, après rehydratation, sur les plaies ou appliquées sur un pansement humide.

Il est bien entendu que les modes de mise en oeuvre décrits ci-dessus ne sont aucunement limitatifs et pourront donner lieu à toute modification désirable, sans sortir pour cela du cadre de l'invention.

**Revendications**

1 - Procédé de fabrication de structures organisées de collagène d'origine animale, caractérisé par la succession des étapes suivantes:

a) on lave et on aseptise un tissu animal fragmenté (1; 101; 201; 301) contenant du collagène pour obtenir un échantillon exempt d'agent contaminant (3; 103; 203; 303);

b) on solubilise au moins une partie du collagène contenu dans l'échantillon (3; 103; 203; 303) obtenu selon a) dans au moins une solution aqueuse aseptique (SA1, SA2, SA3) d'au moins un dérivé diaminé ou un sel de métal alcalin ou alcalino-terreux à un pH compris entre 6,5 et 7,5 et on sépare le résidu non solubilisé (6; 106; 106a; 106b; 206; 206a; 206b) pour obtenir un extrait constitué par une suspension de fibrilles de collagène, extrait que l'on ne conserve que si ladite suspension est exempte de précipité non fibrillaire et si ladite suspension comporte une quantité minimum de fibrilles;

c) on dilue la suspension obtenue selon b) avec de l'eau apyrogène et on y ajoute au moins un catalyseur de reconstitution des structures organisées de collagène pris dans le groupe formé par les sucres monoaminés ayant un poids moléculaire compris entre 180 et 250 et les acides uroniques ou leurs dérivés;

d) on laisse maturer le mélange selon c) pour permettre la reconstitution des structures organisées de collagène;

e) on sépare lesdites structures organisées reconstituées du milieu aqueux, on les lave et on les conditionne pour leur utilisation ultérieure.

2 - Procédé selon la revendication 1, caractérisé par le fait que les dérivés des acides uroniques sont des dérivés lactone.

3 - Procédé selon la revendication 2, caractérisé par le fait que l'on choisit comme catalyseur, dans l'étape c) (16; 116; 216) de la revendication 1, l'acide galacturonique.

4 - Procédé selon la revendication 3, caractérisé par le fait que l'on choisit une quantité d'acide galacturonique telle que l'on obtienne un pH compris entre environ 4 et environ 5.

5 - Procédé selon l'une des revendications 1 à 4, caractérisé par le fait que, dans l'étape c) (16; 116; 216) de la revendication 1, l'on choisit une dilution de la suspension telle que le rapport en volume (suspension initiale/eau de dilution) soit compris entre 1/10 et 1/20.

6 - Procédé selon l'une des revendications 1 à 5, caractérisé par le fait que, dans l'étape c) (16; 116; 216) de la revendication 1, on fait agir le catalyseur à une température comprise entre 30° C et 50° C pendant un temps compris entre 30 et 90 minutes.

7 - Procédé selon l'une des revendications 1 à 6, caractérisé par le fait que, dans l'étape d) (16a; 116a; 216a) de la revendication 1, on laisse maturer ledit mélange au repos à une température comprise entre 1°C et 10°C pendant un temps compris entre 10 heures et 100 heures.

8 - Procédé selon l'une des revendications 1 à 7, caractérisé par le fait qu'on sépare les structures organisées de collagène reconstituées du milieu aqueux par centrifugation (20, 26; 120, 126; 220, 226).

9 - Procédé selon l'une des revendications 1 à 8, caractérisé par le fait qu'on lave plusieurs fois les structures organisées de collagène (22, 27; 122, 127; 222, 227) séparées du milieu aqueux à l'eau apyrogène et à l'éthanol.

10 - Procédé selon la revendication 8, caractérisé par le fait qu'on recycle le liquide centrifugé (21; 121; 221) en y ajoutant le catalyseur de l'étape c) (16; 116; 216) de la revendication 1, en le faisant maturer dans une étape (24a; 124a; 224a) analogue à l'étape d) (16a; 116a; 216a) et en extrayant les structures organisées formées dans une étape (26; 126; 226) analogue à l'étape e) (20; 120; 220).

11 - Procédé selon l'une des revendications 1 à 10, caractérisé par le fait qu'on obtient l'échantillon exempt d'agent contaminant (3; 103; 203; 303) en fractionnant le tissu animal et en lavant au moins une fois les fragments obtenus (1; 101; 201; 301) à l'eau apyrogène et à l'éthanol.

12 - Procédé selon l'une des revendications 1 à 11, caractérisé par le fait que, dans l'étape b) (4; 104; 104a; 104b; 204; 204a; 204b; 304f) de la revendication 1, on solubilise au moins une partie du collagène contenu dans l'échantillon (3; 103; 203; 303) en broyant ledit échantillon dans la solution aqueuse (SA1, SA2, SA3) et en agitant le broyat ainsi obtenu.

13 - Procédé selon l'une des revendications 1 à 12, caractérisé par le fait que l'on sépare par tamisage (5; 105; 105a; 205; 305f) le résidu non solubilisé (6; 106; 106a; 106b; 206; 206a; 206b; 306f) dans l'étape b) (4; 104; 104a; 104b; 204; 204a; 204b; 304f) de la revendication 1.

14 - Procédé selon la revendication 13, caractérisé par le fait qu'on recycle le résidu (6; 106; 106a; 106b; 206; 206a; 206b; 306f) séparé par tamisage (5; 105; 105a; 205; 305f) dans une étape de solubilisation analogue à celle mentionnée pour l'étape b) (4; 104; 104a; 104b; 204; 204a; 204b; 306f) de la revendication 1, la concentration saline de la solution aqueuse (SA1, SA2, SA3) étant augmentée d'une solubilisation à la suivante jusqu'à atteindre la concentration maximale utilisée, le nombre de recyclages étant inférieur à 10 pour chaque solution saline mise en oeuvre et les extraits étant soumis à l'étape c) (16; 116; 216) de la revendication 1.

15 - Procédé selon l'une des revendications 13 ou 14, caractérisé par le fait qu'on lave l'élément de tamisage avec de l'eau apyrogène, qu'on ajoute à cette eau de lavage le catalyseur de l'étape c) (16; 116; 216) de la revendication 1 et qu'enfin, après une étape de maturation (17a; 117a; 217a) analogue à celle de l'étape d) (16a; 116a; 216a) de la revendication 1, on ajoute ce deuxième mélange au mélange maturé de l'étape d) de la revendication 1.

16 - Procédé selon l'une des revendications 1 à 15, dans lequel les structures organisées obtenues sont des fibres de collagène, caractérisé par le fait que l'on utilise, comme tissu animal, de la peau humaine (1).

17 - Procédé selon la revendication 16, caractérisé par le fait que, dans l'étape b) (4) de la revendication 1, on utilise, comme sel pour la solution saline de solubilisation (SA1), au moins un chlorure de métal alcalino-terreux à une concentration comprise entre 0,5 M et 2,5 M.

18 - Procédé selon l'une des revendications 1 à 15, caractérisé par le fait que l'on utilise, comme tissu animal, au moins un constituant de placenta humain (101; 201; 301).

19 - Procédé selon la revendication 18, caractérisé par le fait que l'on choisit, comme constituant de placenta humain, un constituant (101) contenant principalement du collagène de type I et III.

20 - Procédé selon la revendication 19, caractérisé par le fait qu'on effectue l'étape de solubilisation (104; 104a; 104b) selon b) de la revendication 1 successivement avec au moins deux solutions différentes (SA1, SA2, SA3), la première (SA1) contenant au moins un sel de métal alcalin alors que la seconde (SA2) contient au moins un sel de métal alcalino-terreux.

21 - Procédé selon les revendications 14 et 20 prises en combinaison, dans lequel les structures organisées obtenues sont des fibres de collagène, caractérisé par le fait que le résidu (106), obtenu après la dernière solubilisation dans la première solution saline (SA1), est lavé au moins une fois à l'eau apyrogène puis est soumis à la deuxième solution saline (SA2) dans laquelle, par recyclage des résidus non solubilisés (106a), on réalise plusieurs solubilisations successives, tous les extraits obtenus par une solubilisation étant soumis à la catalyse de l'étape c) (116).

22 - Procédé selon les revendications 14 et 21 prises en combinaison, dans lequel les structures organisées obtenues sont des membranes de collagène, caractérisé par le fait que le résidu (106a) obtenu après la dernière solubilisation dans la deuxième solution saline (SA2) est lavé au moins une fois à l'eau apyrogène puis est soumis à une troisième solution (SA3), contenant au moins un dérivé diaminé, dans laquelle par recyclage des résidus non solubilisés (106b) on réalise plusieurs solubilisations successives, tous les extraits obtenus par les solubilisations dans la solution saline (SA3) étant soumis à la catalyse de l'étape c).

23 - Procédé selon la revendication 18, dans lequel les structures organisées obtenues sont des membranes de collagène, caractérisé par le fait que l'on choisit, comme constituant de placenta humain, un constituant (201) contenant principalement du collagène de type IV, V ou VI.

24 - Procédé selon la revendication 23, caractérisé par le fait qu'on effectue l'étape de solubilisation (204b) selon b) de la revendication 1 avec au moins une solution (SA3) contenant au moins un dérivé diaminé.

25 - Procédé selon la revendication 24, caractérisé par le fait que dans l'étape de solubilisation (204; 204a) selon b) de la revendication 1 on utilise, préalablement à la solution saline (SA3), successivement deux solutions salines différentes (SA1, SA2), la première (SA1) contenant au moins un sel de métal alcalin alors que la seconde (SA2) contient au moins un sel de métal alcalino-terreux.

26 - Procédé selon la revendication 18, caractérisé par le fait que l'on utilise, comme tissu animal, un placenta humain entier (301).

27 - Procédé selon la revendication 26, caractérisé par le fait qu'on effectue l'étape de solubilisation selon b) de la revendication 1 successivement avec au moins trois solutions différentes (SA3, SA1, SA2), la première (SA3) contenant au moins un dérivé diaminé, la deuxième (SA1) contenant au moins un sel de métal alcalin et la troisième (SA2) contenant au moins un sel de métal alcalino-terreux.

28 - Procédé selon les revendications 14 et 27 prises en combinaison, dans lequel les structures organisées de collagène sont des fibres de collagène, caractérisé par le fait que le résidu obtenu après la dernière solubilisation dans la première solution (SA3), est lavé au moins une fois à l'eau apyrogène, puis est soumis à la deuxième solution (SA1), dans laquelle, par recyclage des résidus non solubilisés, on réalise plusieurs solubilisations success ives, et que le résidu obtenu après la dernière solubilisation dans la deuxième solution (SA1) est lavé au moins une fois à l'eau apyrogène, puis est soumis à la troisième solution (SA2), dans laquelle par recyclage des résidus non solubilisés, on réalise plusieurs solubilisations successives, la catalyse de l'étape c) de la revendication 1 étant appliquée au moins à tous les extraits obtenus par les solubilisations dans les solutions (SA1, SA2).

29 - Procédé selon les revendications 14 et 28 prises en combinaison, dans lequel les structures organisées obtenues sont des membranes de collagène, caractérisé par le fait que le résidu obtenu après la dernière solubilisation dans la troisième solution (SA2) est lavé au moins une fois à l'eau apyrogène, puis est soumis à une quatrième solution (SA3) contenant au moins un dérivé diaminé, dans laquelle, par recyclage des résidus non solubilisés, on réalise plusieurs solubilisations successives, tous les extraits obtenus par les solubilisations dans la quatrième solution (SA3) étant soumis à la catalyse de l'étape c) de la revendication 1.

30 - Procédé selon l'une des revendications 17, 20, 25 ou 27, caractérisé par le fait que l'on choisit, comme sel de métal alcalino-terreux, le chlorure de calcium à une concentration comprise entre 0,5 M environ et 1,5 M environ.

31 - Procédé selon l'une des revendications 20, 25 ou 27, caractérisé par le fait qu'on choisit, comme sel de métal alcalin, le chlorure de sodium à une concentration comprise entre 0,5 M environ et 2 M environ.

32 - Procédé selon l'une des revendications 22, 24, 28 ou 29, caractérisé par le fait qu'on choisit, le dérivé diaminé dans le groupe formé par la guanidine, l'urée et leurs sels et on utilise une concentration pour la solution de solubilisation (SA3) comprise entre environ 2 M et environ 6 M.

33 - Procédé selon l'une des revendications 1 à 32, caractérisé par le fait que l'on conditionne les structures organisées obtenues par lyophilisation (33; 133; 233) et stérilisation (34; 134; 234).

34 - Procédé selon la revendication 33, caractérisé par le fait que l'on stérilise les structures organisées lyophilisées par irradiation électronique.

35 - Fibres de collagène (36) caractérisées par le fait que:
- elles sont d'origine humaine;
- elles sont à l'état isolé, séparées de leur tissu cellulaire d'origine, constituées en réseau (35) et prêtes à l'emploi;
- elles ont une longueur comprise entre 0,1 mm et 4 mm;
- elles ont une dimension transversale comprise entre 100 nm et 500 nm;
- elles ont une périodicité de striations transversales (37) comprise entre 68 nm et 72 nm;

36 - Structures organisées de collagène caractérisées par le fait qu'elles sont obtenues par le procédé selon l'une des revendications 1 à 34.

FIG. 1

0297972

FIG. 2

0297972

FIG. 3

0297972

FIG. 4

0297972

FIG. 5